(19) Europäisches Patentamt
European Patent Office
Office européen des brevets

(11) Veröffentlichungsnummer: **0 563 733 A1**

# (12) EUROPÄISCHE PATENTANMELDUNG

(21) Anmeldenummer: **93104661.9**

(51) Int. Cl.[5]: **A61K 31/535**, A61K 35/18

(22) Anmeldetag: **22.03.93**

(30) Priorität: **02.04.92 DE 4210939**

(43) Veröffentlichungstag der Anmeldung:
**06.10.93 Patentblatt 93/40**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Neumann, Rainer, Dr.**
**Albert-Einstein-Strasse 27**
**W-5024 Pulheim 2(DE)**
Erfinder: **Schriewer, Michael, Dr.**
**Am Thelen Siefen 1a**
**W-5068 Odenthal(DE)**
Erfinder: **Grohe, Klaus, Dr.**
**Am Wasserturm 10**
**W-5068 Odenthal(DE)**
Erfinder: **Hagemann, Hermann, Dr.**
**Kandinskystrasse 52**
**W-5090 Leverkusen 1(DE)**
Erfinder: **Paessens, Arnold, Dr.**
**Stresemannstrasse 51**
**W-5657 Haan(DE)**
Erfinder: **Reefschläger, Jürgen, Dr.**
**Kranichweg 14**
**W-5600 Wuppertal 1(DE)**
Erfinder: **Streissle, Gert, Dr.**
**Gellertweg 22**
**W-5600 Wuppertal 1(DE)**

## (54) Verwendung von 7-Oxo-7H-pyrido 1,2,3-de 1,4 Benzoxacin-6-carbonsäuren und Estern zur Herstelellung eines Arzneimittels zur Behandlung viraler Krankheiten.

(57) Die vorliegende Erfindung betrifft die Verwendung von bekannten 7-Oxo-7H-pyrido[1,2,3-de][1,4]benzoxacin-6-carbonsäuren und -estern als antiviral wirksame Arzneimittel.

EP 0 563 733 A1

Die vorliegende Erfindung betrifft die Verwendung von bekannten 7-Oxo-7H-pyrido[1,2,3-de][1,4]-benzoxacin-6-carbonsäuren und -estern als antiviral wirksame Arzneimittel.

Die erfindungsgemäß zu verwendenden Verbindungen sind entweder als antibakterielle Wirkstoffe oder als Zwischenprodukte zu deren Herstellung aus der EP-A-253 235 bekannt.

Es wurde nun überraschenderweise gefunden, daß die nachfolgend aufgeführten Verbindungen
9,10-Difluor-3-methyl-7-oxo-7H-pyrido[1,2,3-de]-[1,4]benzoxacin-6-carbonsäure 9-Fluor-3-methyl-10-(4-methyl-1-piperazinyl)-7-oxo-7H-pyrido[1,2,3-de]-[1,4]benzoxacin-6-carbonsäure
9-Fluor-3-methyl-10-(1-piperazinyl)-7-oxo-7H-pyrido[1,2,3-de]-[1,4]benzoxacin-6-carbonsäure
9-Fluor-3-methyl-10-(3-methyl-1-piperazinyl)-7-oxo-7H-pyrido[1,2,3-de]-[1,4]benzoxacin-6-carbonsäure
9-Fluor-10-(4-methyl-1-piperazinyl)-7-oxo-7H-pyrido[1,2,3-de]-[1,4]benzoxacin-6-carbonsäure
9,10-Difluor-2-methyl-7-oxo-7H-pyrido[1,2,3-de]-[1,4]benzoxacin-6-carbonsäureethylester
9,10-Difluor-2-methyl-7-oxo-7H-pyrido[1,2,3-de]-[1,4]benzoxacin-6-carbonsäure 9-Fluor-2-methyl-10-(4-methyl-1-piperazinyl)-7-oxo-7H-pyrido[1,2,3-de]-[1,4]benzoxacin-6-carbonsäure
9,10-Difluor-7-oxo-2-phenyl-7H-pyrido[1,2,3-de]-[1,4]benzoxacin-6-carbonsäureethylester
9,10-Difluor-7-oxo-2-phenyl-7H-pyrido[1,2,3-de]-[1,4]benzoxacin-6-carbonsäure 6,7-Difluor-9-oxo-9H-cyclohexa[1,2-b]pyrido-[1',2',3'-de][1,4]benzoxacin-10-carbonsäureethylester
eine starke antivirale Wirkung besitzen, und somit geeignet sind zur Verwendung bei der Bekämpfung von viralen Erkrankungen, insbesondere der Hepatitis B.

Bevorzugt verwendet werden
9-Fluor-3-methyl-10-(4-methyl-1-piperazinyl)-7-oxo-7H-pyrido[1,2,3-de]-[1,4]benzoxacin-6-carbonsäure
9-Fluor-3-methyl-10-(1-piperazinyl)-7-oxo-7H-pyrido[1,2,3-de]-[1,4]benzoxacin-6-carbonsäure
9-Fluor-3-methyl-10-(3-methyl-1-piperazinyl)-7-oxo-7H-pyrido[1,2,3-de]-[1,4]benzoxacin-6-carbonsäure
9-Fluor-10-(4-methyl-1-piperazinyl)-7-oxo-7H-pyrido[1,2,3-de]-[1,4]benzoxacin-6-carbonsäure
9-Fluor-2-methyl-10-(4-methyl-1-piperazinyl)-7-oxo-7H-pyrido[1,2,3-de]-[1,4]benzoxacin-6-carbonsäure
9,10-Difluor-2-methyl-7-oxo-7H-pyrido[1,2,3-de]-[1,4]benzoxacin-6-carbonsäureethylester
9,10-Difluor-7-oxo-2-phenyl-7H-pyrido[1,2,3-de]-[1,4]benzoxacin-6-carbonsäureethylester
9,10-Difluor-7-oxo-2-phenyl-7H-pyrido[1,2,3-de]-[1,4]benzoxacin-6-carbonsäure bei der Bekämpfung von viralen Erkankungen, insbesondere der Hepatitis B.

Besonders bevorzugt verwendet werden
9-Fluor-3-methyl-10-(4-methyl-1-piperazinyl)-7-oxo-7H-pyrido[1,2,3-de]-[1,4]benzoxacin-6-carbonsäure
9-Fluor-3-methyl-10-(1-piperazinyl)-7-oxo-7H-pyrido[1,2,3-de][1,4]benzoxacin-6-carbonsäure
9-Fluor-3-methyl-10-(3-methyl-1-piperazinyl)-7-oxo-7H-pyrido[1,2,3-de]-[1,4]benzoxacin-6-carbonsäure
9,10-Difluor-2-methyl-7-oxo-7H-pyrido[1,2,3-de]-[1,4]benzoxacin-6-carbonsäureethylester
bei der Bekämpfung von viralen Erkrankungen, insbesondere der Hepatitis B.

Die oben aufgeführten erfindungsgemäßen Verbindungen können nach den Verfahren, die in der EP-A-253 235 publiziert sind, hergestellt werden.

Der Nachweis wurde in mit Hepatitis B Virus DNA transfizierten Hepatomzellen (HEPG 2.2.15) geführt. Die Ergebnisse der unten aufgeführten Beispiele wurden zu dem in der folgenden Literaturangabe [Sells, M.A.; Chen, M.L., Acs, G., Proc. Natl. Acad. Sci. USA S. 1005 - 1009, Vol. 84 (1987] beschriebenen HBV-Testsystem ermittelt:

Transfizierte Hepatomzellen (HEP G2.2.15) wurden mit verschiedenen Konzentrationen an der jeweiligen Verbindung inkubiert. Durch Behandlung der transfizierten Hepatomzellinie HEP G2.2.15 mit den erfindungsgemäßen Verbindungen konnte das Auftreten der virusspezifischen HBV-DNA im Überstand sowie eine Reduktion des $HB_sAg$ Spiegels gezeigt werden. Im Überstand der Zellkulturen wurde nach PEG Fällung der Gehalt an HBV-DNA bestimmt. Dies geschah unter Verwendung einer nicht radioaktiv markierten HBV genomischen DNA Probe [Pauly, P., 1982, Ph.D. Thesis, University of Göttingen, F.R.G.; Köchel et al., 1990, EMBL, Accession-Nr. X 51790]. Es wurde eine Reduktion sowohl der extrazellulären als auch der HBV spezifischen replikativen Intermediate beobachtet. Gleichzeitig erfolgte der Nachweis der Beeinflussung der $HB_sAG$ Bildung mittels eines kommerziell erhältlichen Elisa Tests. Die angegebenen $IC_{50}$-Werte beziehen sich auf die Substratkonzentrationen, die unter den oben aufgeführten Testbedingungen eine 50%ige Hemmung der HBV-DNA Konzentration im Überstand bewirken.

Tabelle A

| Bsp. Nr. | $IC_{50}$(µM) | SI |
|---|---|---|
| 2 | 10 | ~5 |
| 3 | 0,1 | >100 |
| 4 | 0,1 | 50 |
| 6 | 0,5 | ~100 |

Gleichzeitig erfolgte die Bestimmung des möglicherweise vorhandenen cytotoxischen Einflusses der erfindungsgemäßen Verbindungen mittels Kristallviolettfärbung bzw. über den Einbau von radioaktiv markiertem Thymidin in die zelluläre DNA.

Die Wirkung der erfindungsgemäßen Verbindungen auf andere Zellen wurde durch Inkubation von HEL-, MEF-Zellen getestet. Es zeigte sich keine Beeinflussung der Vitalität dieser allen bis zu einer Konzentration von 250 µM.

Als Indikationsgebiete für die erfindungsgemäß verwendbaren Verbindungen können beispielsweise genannt werden:

Die Behandlung von akuten und chronischen Virusinfektionen, die zu einer Hepatitis führen können, beispielsweise die Infektionen mit Hepatitisviren; ferner Virusinfektionen mit Herpesviren Typ 1, 2, Zytomegaloviren, Varizella-Zoster-Viren und HIV.

Besonders bevorzugt ist die Behandlung von chronischen Hepatitis B Virusinfektionen und die Behandlung von akuter Hepatitis-B Virusinfektion.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Die erfindungsgemäßen Wirkstoffe sollen in den oben aufgeführten pharmazeutischen Zubereitungen, vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Ausgangsverbindungen

Beispiel I

3-(1,1-Dimethoxy-2-propylamino)-2-(2,3,4,5-tetrafluorbenzoyl)acrylsäure-ethylester

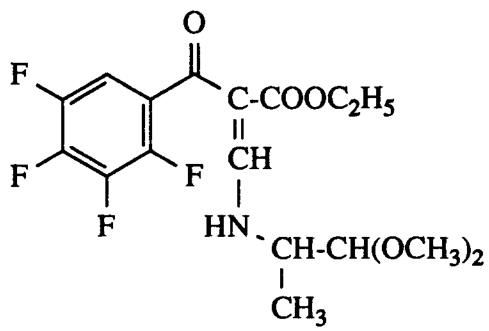

6,4 g 3-Ethoxy-2-(2,3,4,5-tetrafluorbenzoyl)-acrylsäureethylester werden in 8 ml Ethanol vorgelegt. Unter Eiskühlung wird eine Lösung von 2,6 g 1-Amino-propion-aldehyddimethylacetal in 15 ml Ethanol zugetropft. Man läßt auf Raumtemperatur kommen, rührt zwei Stunden und engt dann im Vakuum ein. Ausbeute: 8 g (Öl)

Beispiel II

1-(1,1-Dimethoxy-2-propyl)-4-oxo-6,7,8-trifluor-4H-chinolin-3-carbonsäure-ethylester

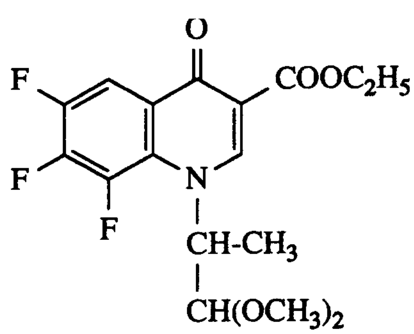

64 g Rohprodukt aus Beispiel I und 24 g $K_2CO_3$ werden in 370 ml DMF vier Stunden auf 140 °C erhitzt. Anschließend gießt man auf Eis. Der Niederschlag wird in $CH_2Cl_2$ aufgenommen. Die organische Phase wird gewaschen und über $Na_2SO_4$ getrocknet. Nach Einengen im Vakuum werden 50 g eines schmierigen Feststoffs erhalten, der durch Waschen mit Ether gereinigt wird. Ausbeute: 42 g F °C : 125-126

Beispiel III

4-Oxo-1-(1-oxo-2-propyl)-6,7,8-trifluor-4H-chinolin-3-carbonsäure

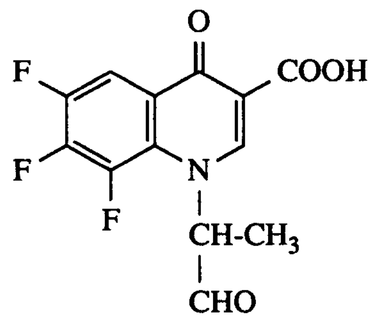

4,4 g Produkt aus Beispiel II werden zusammen mit 15 ml Essigsäure, 13 ml Wasser und 1,3 ml Schwefelsäure vier Stunden auf 140 °C erhitzt. Nach Abkühlung auf Raumtemperatur gibt man Wasser zu und isoliert den ausgefallenen Feststoff. Ausbeute: 3,0g
Schmp.: 188-190 °C (Zers.)

Beispiel IV

2-(2,3,4,5-Tetrafluorbenzoyl)-3-(2,2-dimethoxy-ethylamino)acrylsäure-ethylester

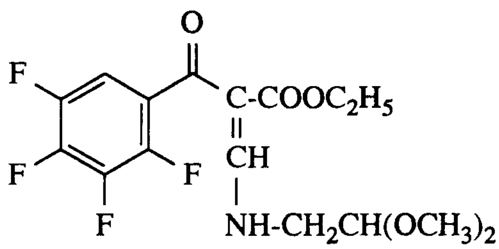

Analog zu Beispiel I wird durch Umsetzung von 3-Ethoxy-2-(2,3,4,5-tetrafluorbenzoyl)-acrylsäureethylester mit Aminoacetaldehyd-dimethylacetal die Titelverbindung erhalten (Öl).

Beispiel V

1-(2,2-Dimethoxyethyl)-4-oxo-6,7,8-trifluor-4H-chinolin-3-carbonsäureethylester

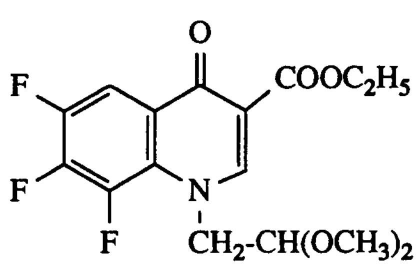

Die Cyclisierung des Produktes aus Beispiel IV erfolgt analog der Umsetzung aus Beispiel II und ergibt die Titelverbindung.

Schmp.: 104-106 ° C

Beispiel VI

1,4-Dihydro-4-oxo-1-(2-oxo-ethyl)-6,7,8-trifluor-4H-chinolin-3-carbonsäure

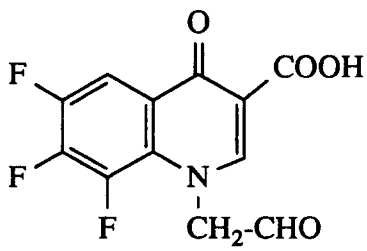

Die Verseifung des Produktes aus Beispiel V in Analogie zu Beispiel III ergibt die Titelverbindung. Schmelzpunkt 220-2 ° C.

Herstellungsbeispiele

Beispiel 1

9,10-Difluor-3-methyl-7-oxo-7H-pyrido[1,2,3-de]-[1,4]benzoxacin-6-carbonsäure

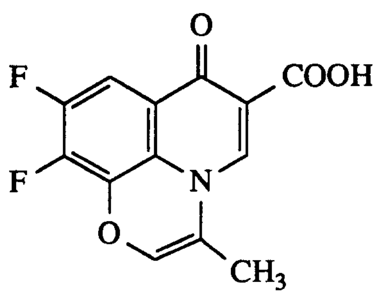

2,2 g Produkt aus Beispiel III werden mit 0,6 g NaOH in einem Gemisch aus 20 ml Wasser und 20 ml Ethanol vier Stunden gekocht. Nach Abkühlung auf Raumtemperatur wird mit verdünnter Salzsäure angesäuert und der entstandene Feststoff isoliert.
Ausbeute: 1,8g
Schmp.: 248-250 ° C (Zers.)

Beispiel 2

9-Fluor-3-methyl-10-(4-methyl-1-piperazinyl)-7-oxo-7H-pyrido[1,2,3-de]-[1,4]benzoxacin-6-carbonsäure

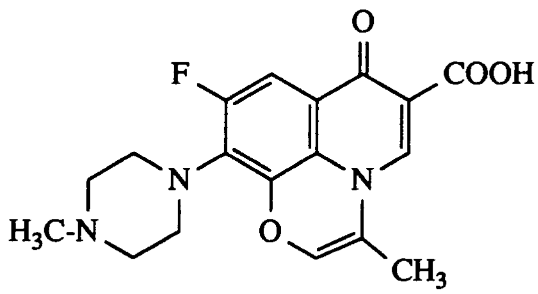

1,6 g Produkt aus Beispiel 1 und 2,9 g N-Methyl-piperazin werden in 20 ml DMSO 2,5 Stunden auf 140 °C erhitzt. Nach Abkühlung wird im Hochvakuum eingeengt. Der Rückstand wird mit Wasser vesetzt. Den Niederschlag isoliert man und trocknet ihn im Vakuum bei 50 °C.
Ausbeute: 0,4g
Schmp.: 262-263 °C (Zers.)
Analog zu Beispiel 2 werden die in Tabelle 1 aufgeführten Verbindungen hergestellt:

Tabelle 1:

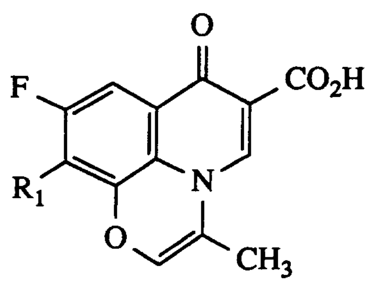

| Bsp.-Nr. | $R^1$ | F°C |
|---|---|---|
| 3 | 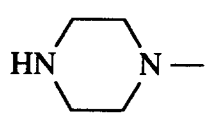 | 270-272 (Zers.) |
| 4 | 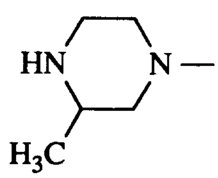 | 190-194 |

Beispiel 5

9-Fluor-10-(4-methyl-1-piperazinyl)-7-oxo-7H-pyrido[1,2,3-de[[1,4]benzoxazin-5-carbonsäure

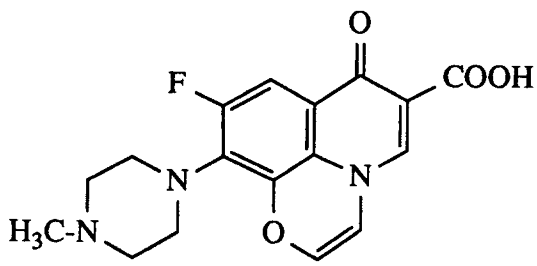

2,85 g 4-Oxo-1-(2-oxoethyl)-6,7,8-trifluor-4H-chinolin-3-carbonsäure und 5,0 g N-Methylpiperazin werden in 30 ml DMSO 2,5 Stunden auf 140 °C erhitzt. Anschließend wird alles Flüchtige im Hochvakuum entfernt. Der Rückstand wird mit Acetonitril verrührt. Der Feststoff wird isoliert und getrocknet. Ausbeute: 2,6 g Schmp.: >300 °C

Beispiel 6

9,10-Difluor-2-methyl-7-oxo-7H-pyrido[1,2,3-de][1,4]benzoxacin-6-carbonsäureethylester

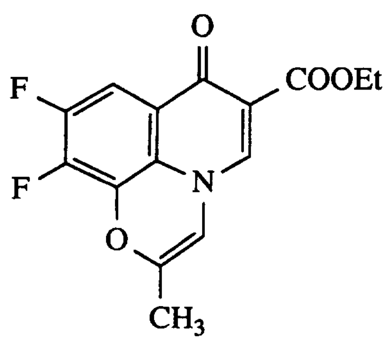

5 g 6,7,8-Trifluor-4-hydroxy-3-chinolincarbonsäureethylester, 3,5 g Chloraceton und 6,6 g $K_2CO_3$ werden in 40 ml DMF zehn Stunden auf 90 °C erhitzt. Nach Abkühlen wird im Vakuum alles Flüchtige abgezogen. Der Rückstand wird mit Wasser verrührt. Der zurückbleibende Feststoff wird isoliert, getrocknet und aus DMF umkristallisiert.
Ausbeute: 3,4g
Schmp.: 229-230 °C

Beispiel 7

9,10-Difluor-2-methyl-7-oxo-7H-pyrido[1,2,3-de][1,4]benzoxacin-6-carbonsäure

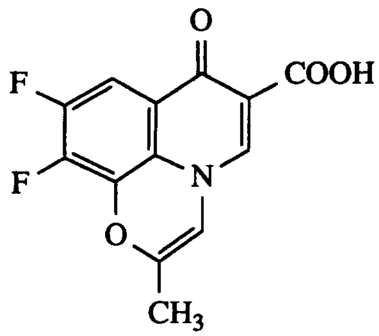

In Analogie zur Vorschrift des Beispiel 6 wird die Titelverbindung erhalten. Ausbeute: 2,4g
Fp.: >300 °C

Beispiel 8

9-Fluor-2-methyl-10-(4-methyl-1-piperazinyl)-7-oxo-7H-pyrido[1,2,3-de][1,4]benzoxacin-6-carbonsäure

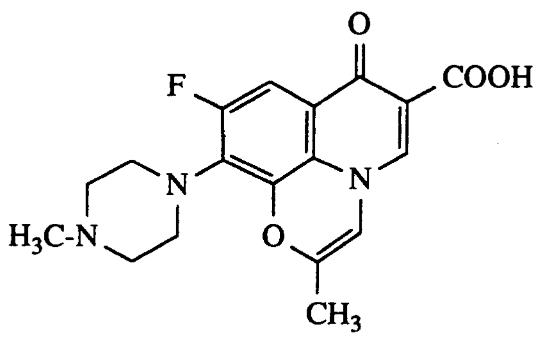

2,4 g Produkt aus Beispiel 7 und 4,3 g N-Methylpiperazin werden in 25 ml DMSO 2,5 Stunden auf 140 °C erhitzt. Anschließend wird alles Flüchtige im Vakuum abgezogen. Der Rückstand wird mit Wasser verrührt. Der verbleibende Feststoff wird isoliert und getrocknet.
Ausbeute: 1,3g
Schmp.: 255-256 °C (Zers.)

Beispiel 9

9,10-Difluor-7-oxo-2-phenyl-7H-pyrido[1,2,3-de][1,4]benzoxacin-6-carbonsäureethylester

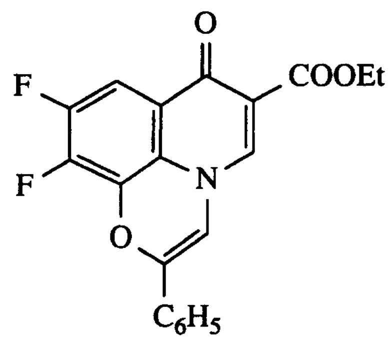

5 g 6,7,8-Trifluor-4-hydroxy-3-chinolincarbonsäureethylester, 5,9 g ω-Chloracetophenon und 6,6 g $K_2CO_3$ werden in 40 ml DMF 10 Stunden auf 90 °C erhitzt. Anschließend wird im Vakuum eingeengt. Den Rückstand verrührt man mit Wasser, isoliert den Feststoff und kristallisiert ihn aus DMF um. Ausbeute: 2,4g Schmp.: 251-252 °C

Beispiel 10

9,10-Difluor-7-oxo-2-phenyl-7H-pyrido[1,2,3-de][1,4]benzoxacin-6-carbonsäure

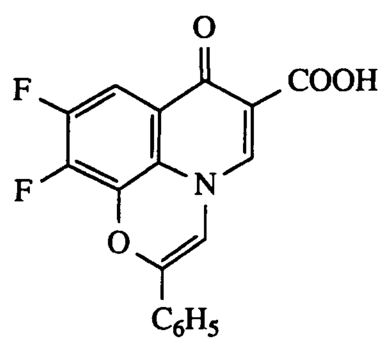

1,9 g Produkt aus Beispiel 9, 6 ml Essigsäure, 4 ml Wasser und 0,5 ml Schwefelsäure werden 4 Stunden gekocht. Danach wird mit Wasser verdünnt und der Feststoff wird abgetrennt.
Ausbeute: 1,7 g
Schmp.: >300 °C

Beispiel 11

6,7-Difluor-9-oxo-9H-cyclohexa[1,2-b] pyrido[1',2',3'-de][1,4]benzoxazin-10-carbonsäureethylester

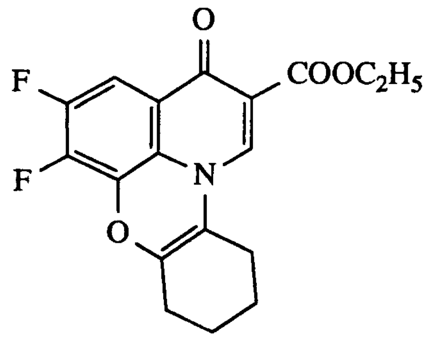

5 g 6,7,8-Trifluor-4-hydroxy-3-chinolincarbonsäureethylester, 5 g 2-Chlorcyclohexanon und 6,6 g $K_2CO_3$ werden in 40 ml DMF 10 Stunden auf 90 ° C erhitzt. Danach engt man im Vakuum ein. Der Rückstand wird in Wasser aufgenommen und mit $CH_2Cl_2$ extrahiert. Die organische Phase wird getrocknet und eingedampft. Den Rückstand trennt man mit Hilfe einer Säule (Kieselgel, Laufmittel, Toluol / Essigester 1:1).
Ausbeute 0,6 g
Schmelzpunkt 198-208 ° C.

## Patentansprüche

1. Verbindungen aus der Gruppe bestehend aus
9,10-Difluor-3-methyl-7-oxo-7H-pyrido[1,2,3-de]-[1,4]benzoxacin-6-carbonsäure
9-Fluor-3-methyl-10-(4-methyl-1-piperazinyl)-7-oxo-7H-pyrido[1,2,3-de]-[1,4]benzoxacin-6-carbonsäure
9-Fluor-3-methyl-10-(1-piperazinyl)-7-oxo-7H-pyrido[1,2,3-de]-[1,4]benzoxacin-6-carbonsäure
9-Fluor-3-methyl-10-(3-methyl-1-piperazinyl)-7-oxo-7H-pyrido[1,2,3-de]-[1,4]benzoxacin-6-carbonsäure
9-Fluor-10-(4-methyl-1-piperazinyl)-7-oxo-7H-pyrido[1,2,3-de]-[1,4]benzoxacin-6-carbonsäure
9,10-Difluor-2-methyl-7-oxo-7H-pyrido[1,2,3-de]-[1,4]benzoxacin-6-carbonsäureethylester
9,10-Difluor-2-methyl-7-oxo-7H-pyrido[1,2,3-de]-[1,4]benzoxacin-6-carbonsäure
9-Fluor-2-methyl-10-(4-methyl-1-piperazinyl)-7-oxo-7H-pyrido[1,2,3-de]-[1,4]benzoxacin-6-carbonsäure
9,10-Difluor-7-oxo-2-phenyl-7H-pyrido[1,2,3-de]-[1,4]benzoxacin-6-carbonsäureethylester
9,10-Difluor-7-oxo-2-phenyl-7H-pyrido[1,2,3-de]-[1,4]benzoxacin-6-carbonsäure
6,7-Difluor-9-oxo-9H-cyclohexa[1,2-b]pyrido-[1',2',3'-de][1,4]benzoxacin-10-carbonsäureethylester zur Bekämpfung von viralen Erkrankungen.

2. Verbindungen aus der Gruppe bestehend aus
9-Fluor-3-methyl-10-(4-methyl-1-piperazinyl)-7-oxo-7H-pyrido[1,2,3-de]-[1,4]-benzoxacin-6-carbonsäure
9-Fluor-3-methyl-10-(1-piperazinyl)-7-oxo-7H-pyrido[1,2,3-de]-[1,4]benzoxacin-6-carbonsäure
9-Fluor-3-methyl-10-(3-methyl-1-piperazinyl)-7-oxo-7H-pyrido[1,2,3-de]-[1,4]benzoxacin-6-carbonsäure
9-Fluor-10-(4-methyl-1-piperazinyl)-7-oxo-7H-pyrido[1,2,3-de]-[1,4]benzoxacin-6-carbonsäure
9-Fluor-2-methyl-10-(4-methyl-1-piperazinyl)-7-oxo-7H-pyrido[1,2,3-de]-[1,4]benzoxacin-6-carbonsäure
9,10-Difluor-2-methyl-7-oxo-7H-pyrido[1,2,3-de]-[1,4]benzoxacin-6-carbonsäureethylester
9,10-Difluor-7-oxo-2-phenyl-7H-pyrido[1,2,3-de]-[1,4]benzoxacin-6-carbonsäureethylester
9,10-Difluor-7-oxo-2-phenyl-7H-pyrido[1,2,3-de]-[1,4]benzoxacin-6-carbonsäure zur Bekämpfung von viralen Erkankungen.

3. Verbindungen aus der Gruppe bestehend aus
9-Fluor-3-methyl-10-(4-methyl-1-piperazinyl)-7-oxo-7H-pyrido[1,2,3-de]-[1,4]benzoxacin-6-carbonsäure
9-Fluor-3-methyl-10-(1-piperazinyl)-7-oxo-7H-pyrido[1,2,3-de][1,4]benzoxacin-6-carbonsäure
9-Fluor-3-methyl-10-(3-methyl-1-piperazinyl)-7-oxo-7 H-pyrido[1,2,3-de]-[1,4]benzoxacin-6-carbonsäure
9,10-Difluor-2-methyl-7-oxo-7H-pyrido[1,2,3-de]-[1,4]benzoxacin-6-carbonsäureethylester zur Bekämpfung von viralen Erkrankungen.

4. Verbindungen gemäß Ansprüchen 1 bis 3 zur Bekämpfung der Hepatitis B.

5. Verwendung von Verbindungen gemäß Ansprüchen 1 bis 3 zur Herstellung von antiviralen Arzneimitteln.

6. Verwendung von Verbindungen gemäß Ansprüchen 1 bis 3 zur Herstellung von Arzneimitteln zur Bekämpfung der Hepatitis B.

7. Antivirale Mittel enthaltend mindestens eine Verbindung gemäß Ansprüchen 1 bis 3.

8. Arzneimittel zur Bekämpfung der Hepatitis B enthaltend mindestens eine Verbindung gemäß Ansprüche 1 bis 3.

Europäisches Patentamt

# EUROPÄISCHER RECHERCHENBERICHT

Nummer der Anmeldung

EP 93 10 4661

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X<br>A | EP-A-0 253 235 (BAYER AG)<br>* Seite 11, Zeile 1 - Seite 21, Zeile 22 *<br>----- | 1-5,7,8<br>6 | A61K31/535 |
| | | | **RECHERCHIERTE SACHGEBIETE (Int. Cl.5 )** |
| | | | A61K |

Der vorliegende Recherchenbericht wurde für alle Patentansprüche erstellt

| Recherchenort | Abschlußdatum der Recherche | Prüfer |
|---|---|---|
| MUENCHEN | 17 MAI 1993 | TZSCHOPPE D. A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)